# EUROPÄISCHE PATENTANMELDUNG

(11) **EP 4 707 842 A1**
(43) Veröffentlichungstag der Anmeldung: **11.03.2026**
(21) Anmeldenummer: 24199110.8
(22) Anmeldetag: 09.09.2024
(51) Int. Cl.: G01R 33/28

(54) **MODULARE VIDEOWIEDERGABEEINRICHTUNG FÜR EINEN PATIENTEN AUF EINER PATIENTENLIEGE IN EINER MAGNETRESONANZEINRICHTUNG UND MAGNETRESONANZEINRICHTUNG**

(71) Anmelder: Siemens Healthineers AG, 91301 Forchheim (DE)
(72) Erfinder: Kelber, Vincent, 96050 Bamberg (DE); Matschl, Volker, 96049 Bamberg (DE); Sukkau, Johann, 91074 Herzogenaurach (DE); Weber, Hans, 91054 Erlangen (DE)
(74) Vertreter: Siemens Healthineers Patent Attorneys

(57) **Zusammenfassung**

Die Erfindung betrifft eine modulare Videowiedergabeeinrichtung (6) für einen Patienten auf einer Patientenliege (5) in einer Magnetresonanzeinrichtung (1), aufweisend
- wenigstens ein Anzeigemodul (9) mit jeweils einer Anzeigeeinheit für ein linkes und ein rechtes Auge (25) eines Kopfes (15) des Patienten, wobei jede Anzeigeeinheit umfasst eine Displayeinrichtung (27),
eine Trägeranordnung (26), um die Displayeinrichtung (27) in einer von dem Kopf (15), insbesondere seitlich, beabstandeten Position bei in einer Verwendungsposition befindlicher Anzeigeeinheit zu haltern, und
eine an der Trägeranordnung (26), insbesondere wenigstens teilweise vor dem jeweiligen Auge (25), befestigte Optikanordnung (31) zur Projektion eines von der Displayeinrichtung (27) ausgegebenen Bildes in der Verwendungsposition zu dem Auge (25),

- wenigstens eine Halterung (10), die mit dem Anzeigemodul (9) lösbar verbindbar ist und Befestigungsmittel zur lösbaren Befestigung der Halterung (10) wenigstens an einer auf der Patientenliege (5) angeordneten und/oder anordenbaren Komponente (7) aufweist,
- eine mit dem Anzeigemodul (9) verbindbare Datenübertragungsanordnung (12), und
- eine mit dem Anzeigemodul (9) verbindbare Stromversorgungsanordnung (11).

## Beschreibung

Die Erfindung betrifft eine modulare Videowiedergabeeinrichtung für einen Patienten auf einer Patientenliege in einer Magnetresonanzeinrichtung und eine Magnetresonanzeinrichtung.

Patientenaufnahmen von Magnetresonanzeinrichtungen zur medizinischen Bildgebung sind häufig relativ eng. Zudem herrscht innerhalb der Patientenaufnahme während des Aufnahmevorgangs eine hohe Lärmbelastung und Aufnahmevorgänge bei Magnetresonanzeinrichtungen benötigen meist eine längere Zeitdauer, beispielsweise von 10 Minuten bis hin zu einer halben Stunde. Daher wurde im Stand der Technik bereits vorgeschlagen, einen Patienten, der auf einer Patientenliege innerhalb einer Patienten Aufnahme einer Magnetresonanzeinrichtung, insbesondere für einen Aufnahmevorgang, befindlich ist, multimedial zu unterhalten. Beispielsweise ist es bekannt, einen Kopfhörer zu verwenden, der gleichzeitig dem Lärmschutz und der Ausgabe von Audio-Inhalten dient.

Auch hinsichtlich der Ausgabe von Video-Inhalten wurden im Stand der Technik bereits verschiedene Lösungen vorgeschlagen. So ist es beispielsweise bekannt, oberhalb des Patienten in Blickrichtung bei Rückenlagerung einen Spiegel vorzusehen, mit dem die Blickrichtung des Patienten von vertikal auf horizontal ausgerichtet wird, um so einen magnetresonanzkompatiblen Monitor beobachten zu können, der außerhalb der Patientenaufnahme, beispielsweise auf einer hinteren Seite einer Schirmkabine für die Hauptmagneteinheit, platziert wird. Insbesondere kann ein derartiger, beispielsweise in einem 45°-Winkel ausgerichteter Spiegel an einer Kopfspule, in der der Kopf des Patienten befindlich ist, befestigt sein. Eine derartige Ausgestaltung ist beispielsweise in DE 10 2013 217 561 B4 beschrieben. Hierbei besteht allerdings das Problem, dass der Abstand zwischen der Quelle der Bilder, also dem Monitor, und den Augen des Patienten von der Liegenposition abhängig ist. Auf diese Weise hängt auch die Größe des Bildes sehr stark von dieser Liegenposition ab und damit davon, welches Körperteil gerade untersucht wird. Dies ist nachteilig im Hinblick auf die Bildwahrnehmung und die Akkommodation.

Vorgeschlagen wurde ferner ein unter dem Namen "Innovision" bekanntes System, bei dem der insbesondere wiederum mit einem Winkel von 45° ausgerichtete Spiegel eine parabolischer Form aufweist, um so ein Videobild hinter dem Kopf des Patienten zu vergrößern, welches von einem in ein Innovision-Gerät integrierten Videoprojektor erzeugt wird. Hierbei muss ein schweres Gerät innerhalb der Patientenaufnahme verwendet werden. Aufgrund der Höhe des Geräts muss die Höhenfahrt der Patientenliege mechanisch begrenzt werden, damit das Gerät nicht durch ein versehentliches zu weites Hochfahren der Patientenliege und eine Kollision des Geräts mit der Wand der Patientenaufnahme der Hauptmagneteinheit beschädigt wird. Daher wäre ein in der Höhe deutlich kleineres Videosystem wünschenswert.

Vorgeschlagen wurde darüber hinaus, dem Patienten eine Videobrille aufzusetzen, wie sie beispielsweise in DE 10 2007 030 972 B3 vorgeschlagen wurde. Eine Videobrille ist jedoch unhandlich in der Handhabung, da sie von dem Patienten erst angezogen werden muss, was die Vorbereitungszeit einer Magnetresonanzuntersuchung erhöht. Zudem wird der Patient durch die Videobrille von der Außenwelt abgeschnitten, was von einigen Patienten nicht akzeptiert wird. Darüber hinaus ist ein Kabel mit einer Stromversorgung und einer Signalübertragung notwendig, welches in der Vorbereitungszeit angebracht werden muss. Auch hinsichtlich der Magnetresonanz-Kompatibilität können Probleme auftreten. Ein wesentliches Problem ist die Sterilität/Hygiene, da durch den direkten Kontakt mit der Haut des Patienten eine Säuberung nach jedem Patienten erforderlich ist. Hierdurch entsteht Aufwand und es können Komponenten, beispielsweise ein Gummirand, der Videobrille angegriffen werden, was den Verschleiß erhöht. Daher ist ein Gerät ohne direkten Hautkontakt wünschenswert.

Zudem wurden auch in der Umsetzung und dem Aufwand komplexe andere Ansätze vorgeschlagen, beispielsweise die Verwendung eines Bündels aus kohärenten Lichtwellenleitern, wie beispielsweise in US 4,901,141 A beschrieben, und die Nutzung von Relay-Linsen mit Flüssigkristalldisplays, vergleiche beispielsweise US 5,877,732 A.

Ein weiteres Problem mit den bekannten Ansätzen ist, dass sie häufig nicht in allen denkbaren Lagen des Patienten eingesetzt werden können. Beispielsweise können die einen Spiegel, der die Blickrichtung auf einen insbesondere hinter dem Patienten befindlichen Monitor umlenkt, nutzenden Ansätze nur in Rückenlage eingesetzt werden. Bei Anbringung des Spiegels an der Kopfspule ist die Nutzung sogar nur in der Lagerung, in der der Patient mit dem Kopf voran in die Patientenaufnahme eingefahren wird ("head first supine" - HFS), möglich. Hingegen kann die Lösung mit einer Videobrille auch in einer Bauchlage, beispielsweise bei einer Mammographie-Untersuchung, oder in einer Seitenlage, beispielsweise bei einer Prostata-Untersuchung, verwendet werden.

Wünschenswert wäre also ein magnetresonanzkompatibles, leichtes und autarkes Videosystem, dass in möglichst allen Patientenlagerungen eingesetzt werden kann, den Patienten nicht von der Außenwelt komplett abschotten soll, möglichst schnell im Sichtbereich positionierbar sein soll und möglichst ohne direkten Hautkontakt auskommt. Dabei soll Magnetresonanzkompatibilität so verstanden werden, dass die Videowiedergabeeinrichtung möglichst weitgehend unmagnetisch sein soll und keine oder möglichst keine Magnetresonanz-Bildartefakte erzeugen soll. Autarkie bedeutet, dass die Stromversorgung und auch die Datenübertragung möglichst ohne Kabel erfolgen soll. Den Patienten nicht von der Außenwelt abzuschotten soll so verstanden werden, dass dieser zumindest optional seine Umgebung weiterhin wenigstens teilweise wahrnehmen kann. Die Einsetzbarkeit in allen Patientenlagerungen ist so zu verstehen, dass zumindest eine Patientenlagerung auf dem Bauch (Bauchlage), auf der linken und auf der rechten Seite (Seitenlage) und auf dem Rücken (Rückenlage) abgedeckt sein soll. Bei der Rückenlagerung existieren dabei zwei Varianten, nämlich zum einen, dass sich der Kopf in einer Kopfspule befindet und zum anderen, dass sich der Kopf auf einem Kopfkissen befindet. Hierbei ist zu beachten, dass die Kopfspule einen gewissen Bereich über den Augen des Patienten einnimmt, was die Möglichkeiten eines Videosystems einschränkt.

Der Erfindung liegt daher die Aufgabe zugrunde, eine Videowiedergabeeinrichtung für eine Magnetresonanzeinrichtung anzugeben, die möglichst flexibel einsetzbar, kompakt realisierbar und möglichst magnetresonanzkompatibel ist.

Zur Lösung dieser Aufgabe ist erfindungsgemäß eine modulare Videowiedergabeeinrichtung für einen Patienten auf einer Patientenliege in einer Magnetresonanzeinrichtung vorgesehen, welche aufweist
- wenigstens ein Anzeigemodul mit jeweils einer Anzeigeeinheit für ein linkes und ein rechtes Auge eines Kopfes des Patienten, wobei jede Anzeigeeinheit umfasst
   * eine Displayeinrichtung,
   * eine Trägeranordnung, um die Displayeinrichtung in einer von dem Kopf, insbesondere seitlich, beabstandeten Position bei in einer Verwendungsposition befindlicher Anzeigeeinheit zu haltern, und
   * eine an der Trägeranordnung, insbesondere wenigstens teilweise vor dem jeweiligen Auge, befestigte Optikanordnung zur Projektion eines von der Displayeinrichtung ausgegebenen Bildes in der Verwendungsposition zu dem Auge,
      - wenigstens eine Halterung, die mit dem Anzeigemodul lösbar verbindbar ist und Befestigungsmittel zur lösbaren Befestigung der Halterung wenigstens an einer auf der Patientenliege angeordneten und/oder anordenbaren Komponente aufweist,
      - eine mit dem Anzeigemodul verbindbare Datenübertragungsanordnung, und
      - eine mit dem Anzeigemodul verbindbare Stromversorgungsanordnung.

Dabei kann das Anzeigemodul in weniger bevorzugten Ausführungsbeispielen zweiteilig sein, das bedeutet, die Anzeigeeinheiten können beispielsweise getrennt positioniert werden. Bevorzugt jedoch kann vorgesehen sein, dass beide Anzeigeeinheiten in dem einen Anzeigemodul integriert sind. Hierzu kann besonders zweckmäßig vorgesehen sein, dass die Trägeranordnungen beider Anzeigeeinheiten zu einer Trägereinrichtung des Anzeigemoduls mittels eines Verbindungsmittels verbindbar oder verbunden sind. Dies verbessert die Handhabbarkeit und macht sich den Umstand zunutze, dass Augen an einem Kopf üblicherweise in einer relativ festen, vorbekannten räumlichen Relation zueinander angeordnet sind.

Um einen kompakten, möglichst niedrigen Aufbau zu fördern, sind die Displayeinrichtungen in der Verwendungsposition bevorzugt neben dem Kopf des Patienten angeordnet, wobei die Optikanordnungen dem Patienten dennoch den Blick auf diese erlauben. Neben dem Kopf des Patienten existiert meist ein gewisser Freiraum, der genutzt werden kann. Insbesondere bei einer Rückenlage oder einer Bauchlage des Patienten sind hierbei Vorteile gegenüber höher aufbauenden Videosystemen gegeben. Es sind auch weniger bevorzugte Ausführungsformen denkbar, in denen sich die Displayeinrichtungen vor dem Kopf des Patienten befinden. Grundsätzlich ist auch eine Anordnung hinter dem Kopf des Patienten denkbar, wobei dann gegebenenfalls eine aufwändigere Optikanordnung benötigt wird.

In jedem Fall sind die Displayeinrichtungen von dem Kopf des Patienten beabstandet, insbesondere derart, dass keine bzw. eine möglichst geringe Beeinflussung eines Bildgebungsvorgangs mit der Magnetresonanzeinrichtung stattfindet. Mit anderen Worten ermöglicht es die Nutzung der Optikanordnungen, die Displayeinrichtungen weiter vom Kopf des Patienten zu entfernen, um, beispielsweise bei doch noch vorhandenen magnetischen Materialien oder dergleichen, Bildartefakte möglichst weitgehend zu verhindern. Beispielsweise kann vorgesehen sein, dass der Abstand der Displayeinrichtung zu dem Kopf in der Verwendungsposition und insbesondere auch in der Nichtgebrauchsposition wenigstens 5 cm, insbesondere wenigstens 10 cm, beträgt.

Durch den modularen Aufbau ist es problemlos und einfach möglich, beispielsweise beim Wechsel von Lagerungen, die Videowiedergabeeinrichtung umzugestalten, beispielsweise, indem die Halterung entsprechend ausgetauscht wird. Mithin sieht eine besonders vorteilhafte Weiterbildung der vorliegenden Erfindung vor, dass die Videowiedergabeeinrichtung mehrere austauschbare Halterungen für unterschiedlichen Liegepositionen des Patienten zugeordnete Komponenten und/oder Befestigungspositionen an wenigstens einer der wenigstens einen Komponente aufweist. Beispielsweise können also die Halterungen für unterschiedliche Lagerungen des Patienten unterschiedlich ausgelegt sein, beispielsweise zur Befestigung an eine andere Komponente und/oder in ihrer Länge, Ausgestaltung und Form. Durch diese speziell designten Halterungen kann die vorgeschlagene Videowiedergabeeinrichtung in allen Lagerungen des Patienten eingesetzt werden.

Zudem ist die Handhabung für Benutzer angenehmer, weil die Komponenten leicht ausgestaltet werden können, insgesamt kleiner sind und das Gewicht zwischen Teilen, beispielsweise der Stromversorgungsanordnung und der Datenübertragungsanordnung sowie dem Anzeigemodul, aufgeteilt werden kann. Insbesondere kann, wie bereits erwähnt, die vertikale Höhe zumindest bei Rückenlagerung und Bauchlagerung, bevorzugt für alle Lagerungen, eingeschränkt werden, sodass die Einsetzbarkeit erhöht wird und mit weniger Aufwand verbunden ist.

In besonders zweckmäßiger Ausgestaltung der Erfindung ist vorgesehen, dass wenigstens eine der wenigstens einen Halterung eine Bewegungseinrichtung, insbesondere eine Schwenkeinrichtung, zum Bewegen des Anzeigemoduls zwischen der Verwendungsposition und wenigstens einer Nichtgebrauchsposition aufweist. Auf diese Weise kann das Anzeigemodul leicht vor die Augen des Patienten gebracht werden und ebenso leicht dort wieder entfernt werden. Die Nichtgebrauchsposition ist dabei zweckmäßigerweise eine solche, in der das Anzeigemodul und/oder die Halterung an der Patientenliege und/oder der Komponente raumsparend und wenig störend anliegen und/oder sogar wenigstens teilweise versenkt in einer Aufnahme angeordnet sind. Insbesondere kann somit auch bei Verwendung der Videowiedergabeeinrichtung die Vorbereitungszeit des Patienten für einen Untersuchungsvorgang gering gehalten werden. Die Halterung und/oder die Komponente kann, insbesondere seitens der Bewegungseinrichtung, Arretierungsmittel aufweisen, um die Halterung in der Verwendungsposition und/oder der Nichtgebrauchsposition zu arretieren.

Besonders bevorzugt kann der Abstand zwischen der Optikanordnung und dem Auge wenigstens einen Zentimeter, bevorzugt wenigstens 3 cm, insbesondere im Bereich von 1 - 10 cm, betragen und/oder die Optikanordnung und/oder die Trägeranordnung kann wenigstens teilweise transparent ausgebildet sein. Auf diese Weise wird mit besonderem Vorteil erreicht, dass der Patient zumindest teilweise seine Umgebung noch wahrnehmen kann und nicht gänzlich von dieser abgeschottet ist. Durch den Freiraum zwischen der Optikanordnung und dem Auge ist ein Teil des Sichtfelds, insbesondere auch durch die jeweilige Trägeranordnung, nicht blockiert und der Patient fühlt sich weniger beengt. Darüber hinaus kann auch eine zumindest im Blickfeld des Patienten wenigstens teilweise transparente Ausgestaltung der Optikanordnung, beispielsweise die Verwendung eines wenigstens teilweise transparenten Spiegels, diesbezüglich ermöglichen, dass der Patient trotz der Wahrnehmung auf der Displayeinrichtung wiedergegebener Bilder noch Umgebungswahrnehmung besitzt. Somit kann diesbezüglichen Patientenwünschen nachgekommen werden. Es sind auch Weiterbildungen denkbar, in denen, falls es entgegengesetzt der Patientenwunsch ist, völlig abgeschottet zu sein, wenigstens ein optional einsetzbares Abschottungselement als Teil des Anzeigemoduls vorgesehen ist, welches entsprechend eingesetzt werden kann. Beispielsweise kann das Abschattungselement zusätzlich an der Trägeranordnung angebracht werden und/oder hinter die Optik bewegt werden bzw. dort befestigt werden.

Zweckmäßigerweise können die Displayeinrichtungen ein LCD-Display, ein OLED-Display und/oder ein Micro-LED-Display sein oder umfassen. Auf diese Weise werden verfügbare Techniken eingesetzt, die eine kleine, leichte und wenig elektrische Leistung zum Betrieb benötigende Ausgestaltung der Displayeinrichtungen erlauben. Dies trägt weiter zur kleinbauenden, einfach handhabbaren Realisierung bei und ermöglicht eine verbesserte Herstellung der Autarkie.

In bevorzugten Ausgestaltungen kann vorgesehen sein, dass die Optikanordnung wenigstens einen, insbesondere parabolisch geformten, Spiegel und/oder wenigstens eine Linse und/oder wenigstens ein Prisma umfasst. Beispielsweise kann die Optikanordnung entweder aus einer Linse oder aus einem Spiegel oder aus einer Kombination dieser Elemente bestehen. In einer besonders bevorzugten Ausgestaltung ist vorgesehen, dass der Spiegel, insbesondere bei einer seitlich angeordneten Displayeinrichtung, wenigstens teilweise transparent ausgebildet ist. Bei einer seitlichen Anordnung der jeweiligen Displayeinrichtung kann der Spiegel beispielsweise zumindest im Wesentlichen in einem 45°-Winkel ausgerichtet sein. Vorzugsweise wird ein teilweise transparenter, parabolisch geformter Spiegel verwendet, der gleichzeitig einen Blick auf die Umgebung erlaubt und das mit dieser überlagerte, wiedergegebene Bild auf die Netzhaut des Patienten projiziert. Der Spiegel befindet sich vor dem Auge des Patienten und lenkt den optischen Pfad um seinen Ausrichtungswinkel, beispielsweise zumindest im Wesentlichen 45°, auf die Displayeinrichtung um. Bei Ausgestaltungen mit einem Spiegel kann die Linse beispielsweise dazu dienen, korrekt auf das Auge zu fokussieren.

Während in einfachen, aber nicht bevorzugten Ausführungsformen die Datenübertragungsanordnung und die Stromversorgungsanordnung durch wenigstens ein Kabel mit Stecker für das Anzeigemodul gebildet sein können, ggf. sogar integriert durch ein einziges Kabel, ist bevorzugt die Videowiedergabeeinrichtung durch die Stromversorgungseinrichtung und die Datenübertragungseinrichtung in dem Sinne autark ausgebildet, dass keine externe Stromversorgung benötigt wird und die wiederzugebenden Bilder zumindest nicht drahtgebunden, insbesondere nicht mittels eines Kabels, bereitgestellt werden. Auf diese Weise wird die Magnetresonanzkompatibilität erhöht sowie die Installation und die Nutzung vereinfacht.

Vorzugsweise kann die Stromversorgungsanordnung einen magnetresonanzkompatiblem elektrischen Akkumulator und/oder wenigstens ein Photovoltaikmodul aufweisen. Auf diese Weise steht elektrische Leistung zum Betrieb der Displayeinrichtungen und ggf. der Datenübertragungseinrichtung ohne Notwendigkeit einer entsprechenden Verkabelung lokal bereit. Nachdem, wie bereits dargelegt wurde, die Auslegung des Anzeigemoduls und, falls notwendig, auch der Datenübertragungseinrichtung, derart erfolgen kann, dass zu deren Betrieb nur wenig Leistung erforderlich ist, sind derartige einfache, magnetresonanzkompatibel und bauplatzsparend umsetzbare Elemente ausreichend. Der MR-kompatible Akkumulator ist dabei bevorzugt zumindest weitgehend ohne magnetische Komponenten ausgebildet. Derartige wiederaufladbare Batterien sind, wenn auch zu teilweise etwas höheren Preisen, auf dem Markt verfügbar. Insbesondere kann der magnetresonanzkompatible Akkumulator kein Schutzblech aus Edelstahl oder dergleichen, sondern ein Kunststoffschutzgehäuse aufweisen und/oder ein Lithium-Ionen-Akkumulator sein.

Denkbar ist es zusätzlich oder alternativ auch, dass die Datenübertragungsanordnung und/oder die Stromversorgungsanordnung wenigstens ein Kabel und/oder wenigstens einen Stecker umfassen, beispielsweise für die Verbindung wenigstens einer Baueinheit wenigstens einer dieser Anordnungen mit dem Anzeigemodul. Denkbar ist es aber auch, über ein Kabel mit Stecker beispielsweise Strom und/oder wiederzugebende Bilder betreffende Daten von einer entfernten Quelle zu erhalten. Beispielsweise kann der Stecker für einen Spulensteckplatz der Patientenliege ausgebildet sein, um dort elektrische Leistung und/oder Daten zu erhalten.

In besonders vorteilhaften Ausführungsbeispielen kann die Datenübertragungsanordnung eine Schnittstelle zum optischen Empfang wiederzugebender Bilder, insbesondere einen Anschluss für ein Glasfaserkabel und/oder eine Li-Fi-Schnittstelle und/oder eine Infrarotlink-Schnittstelle, aufweisen. Li-Fi (Optische Drahtlosübertragung, Light Fidelity) ist eine optische drahtlose Technologie zur Datenübertragung. Im Gegensatz zu WLAN oder anderen Funktechnologien arbeitet Li-Fi mit dem Spektrum des Lichts - genauer gesagt mit sichtbarem Licht oder Infrarotstrahlung. Die Datenübertragungsanordnung kann also bevorzugt optisch, konkret drahtlos über Li-Fi und/oder Infrarot Link, arbeiten. Dabei ist beim Einsatz der Videowiedergabeeinrichtung in einer Magnetresonanzeinrichtung diese Variante besonders vorteilhaft. Denn bei einer zylindrischen Patientenaufnahme (oft auch als "Bore" bezeichnet) befindet sich der Patient immer derart auf der Patientenliege, dass sich sein Kopf auf der Kopfseite oder auf der Fußseite der Patientenliege und somit zu einer Seite der Patientenaufnahme befindet. Somit existiert immer auf einer der Seiten der Patientenaufnahme ein direkter, freier Blick auf den Kopf des Patienten und somit die Datenübertragungsanordnung (bzw. ein entsprechendes Empfangsteil der Datenübertragungsanordnung). Mindestens eine von Lichtquellen außerhalb der Patientenaufnahme, die entlang der Längsachse der Patientenaufnahme leuchten, kann den Kopf des Patienten also störungsfrei erreichen.

Insofern sieht eine besonders vorteilhafte Ausgestaltung in diesem Zusammenhang vor, dass die Magnetresonanzeinrichtung und/oder eine Schirmkabine für eine Hauptmagneteinheit der Magnetresonanzeinrichtung, die die Patientenaufnahme aufweist, wenigstens eine optische Sendeeinrichtung umfasst. Insbesondere können bei einer zylindrischen Patientenaufnahme beidseitig in einer Richtung entlang einer Längsachse der Patientenaufnahme (z-Richtung) mittels Li-Fi und/oder Infrarot, insbesondere kollimiert und/oder fokussiert, sendende Sendeeinrichtungen vorgesehen sein. Bei einem grundsätzlich außerhalb der Patientenaufnahme verbleibenden Ende der Patientenliege ("Fußende") kann auch eine der Sendeeinrichtungen dort angeordnet werden. Mit anderen Worten bedeutet dies, dass Videodaten, die wiederzugebende Bilder betreffen, beispielsweise durch einen kollimierten Lichtstrahl mit vergleichsweise geringer Intensität zu einem Empfangsteil der Datenübertragungsanordnung im Bereich des Kopfes des Patienten optisch übertragen werden können. Hierfür sind zwei optische Sendeeinrichtungen, je eine auf jeder Seite außerhalb der Hauptmagneteinheit, beispielsweise an den Wänden der Schirmkabine, installiert. Alternativ kann auch auf der Fußseite die Sendeeinrichtung am Fußende der Patientenliege installiert werden. Das Empfangsteil wird entsprechend im Kopfbereich des Patienten positioniert.

Alternativ oder zusätzlich ist es auch denkbar, dass die Datenübertragungsanordnung eine Funkschnittstelle und/oder eine galvanische Schnittstelle zum Empfang wiederzugebender Bilder, insbesondere also entsprechender Videodaten, aufweist. Eine derartige Ausgestaltung ist jedoch weniger bevorzugt.

An dieser Stelle sei angemerkt, dass die Datenübertragungsanordnung auch eine Datenübertragung in beiden Richtungen, also bidirektional, erlauben kann, mithin insbesondere ein Empfangsteil der Datenübertragungsanordnung auch als Sendeteil ausgebildet sein kann. Dies ist insbesondere dann zweckmäßig, wenn die Videowiedergabeeinrichtung und/oder die Komponente weitere Funktionselemente umfassen, beispielsweise Sensorik oder dergleichen, da dann auch Messdaten ausgegeben werden können. Die Videowiedergabeeinrichtung kann aber auch mit Bedienelementen und/oder sonstigen Eingabemitteln für den Patienten versehen werden, um Information des Patienten nach außerhalb der Patientenaufnahme zu übermitteln. Darüber hinaus können auch andere Daten als wiederzugebende Bilder zu der Videowiedergabeeinrichtung übertragen werden, beispielsweise Audiodaten zur Ausgabe mittels eines Teils der Videowiedergabeeinrichtung bildenden und/oder dieser zugeordneten Audioausgabemittels.

Eine bevorzugte Weiterbildung sieht vor, dass die Anzeigeeinheiten, insbesondere wenigstens deren Displayeinrichtungen, und/oder die Stromversorgungsanordnung und/oder die Datenübertragungsanordnung einen Hochfrequenzschirm aufweisen. Um zu vermeiden, dass die Magnetresonanzbildgebung gestört wird, werden die entsprechenden, Störpotenzial aufweisenden Anteile der Videowiedergabeeinrichtung zweckmäßigerweise mit Hochfrequenzschirmen versehen. Dabei kann der bzw. jeder Hochfrequenzschirm wenigstens teilweise aus Kupfer und/oder Aluminium und/oder Kohlenstoff bestehen und/oder wenigstens teilweise eine Gitterstruktur aufweisen. In einer besonders zweckmäßigen Ausgestaltung kann hierbei vorgesehen sein, dass der Hochfrequenzschirm wenigstens teilweise eine Schichtstruktur umfasst, in der zwischen zwei Kohlenstofffaserschichten eine metallische, insbesondere aus Kupfer bestehende Gitterschicht angeordnet ist. Die Maschenweite der Gitterstruktur der Gitterschicht kann hierbei zweckmäßig im Bereich von ein bis vier mm liegen. Auf diese Weise wird gleichzeitig eine hinreichende Hochfrequenzdämpfung erreicht, ohne dass es zu Störungen des Grundmagnetfelds (B0-Feld) und/oder Erwärmung durch Wirbelströme in einem massiven Hochfrequenzschirm kommt.

Ein Spezialfall ergibt sich für die Displayeinrichtungen, nachdem hier der Hochfrequenzschirm die Erkennbarkeit der wiedergegebenen Bilder nicht oder zumindest möglichst wenig beeinflussen soll. In diesem Zusammenhang sieht eine bevorzugte Weiterbildung der Erfindung vor, dass der Hochfrequenzschirm der Displayeinrichtungen eine Displayfläche der jeweiligen Displayeinrichtung überdeckend wenigstens eine mit ITO und/oder Silber und/oder Gold beschichtete Glasschicht aufweist, insbesondere mehrere solche Glasschichten. Mithin wird im Bereich der Displayfläche, also dort, wo die Bilder wiedergegeben werden, ein spezieller Ansatz gewählt, um die Schirmung umzusetzen. Besonders bevorzugt ist es, ITO (Indium-Zinn-Oxid) einzusetzen, insbesondere also den Hochfrequenzschirm an der Vorderfläche der Displayeinrichtungen mit ITO-beschichtetem Glas hochfrequenzdicht auszugestalten. ITO ist leitfähig und transparent für das sichtbare Licht, sodass bereits für die Verwendung bei LCDs zur Ausbildung von Matrixkontakten eingesetzt wurde. Aufgrund dieser Eigenschaften wird es hier mit besonderem Vorteil als Teil des Hochfrequenzschirms eingesetzt. Alternativ können beispielsweise dünne Schichten aus Silber oder Gold verwendet werden, was jedoch weniger bevorzugt ist, da hierdurch die wahrnehmbare Bildqualität eingeschränkt sein kann. Allgemein ist die Beschichtung, bevorzugt das ITO, am Rand der Glasschicht galvanisch mit dem restlichen Hochfrequenzschirm verbunden. Um eine höhere Hochfrequenzdichte zu erreichen, kann in bevorzugten Ausgestaltungen vorgesehen sein, dass mehrere, beispielsweise bis zu 10, beschichtete Glasschichten pro Displayeinrichtung als ein Stapel verwendet werden, wobei wiederum jede Beschichtung entlang des Randes galvanisch mit dem restlichen Hochfrequenzschirm verbunden ist.

In Ausführungsbeispielen kann vorgesehen sein, dass die Stromversorgungsanordnung und die Datenübertragungsanordnung wenigstens teilweise als eine gemeinsame Baueinheit, insbesondere ein Baueinheitmodul, in einem gemeinsamen Gehäuse angeordnet sind. Auf diese Weise kann auch hinsichtlich dieser Anordnungen zumindest bis zu einem gewissen Grad eine Modularisierung erfolgen, beispielsweise in Form einer zumindest einen großen Teil der Stromversorgungsanordnung und der Datenübertragungsanordnung bereitstellenden Baueinheit, die beispielsweise über wenigstens ein, bevorzugt genau ein, Kabel mit dem Anzeigemodul verbunden werden kann. Es sind auch Ausgestaltungen denkbar, in denen mehrere solche Baueinheiten und/oder zusätzlich einzelne Baueinheiten für die Datenübertragungsanordnung und die Stromversorgungsanordnung vorgesehen sind und/oder zumindest teilweise in die bzw. eine Komponente integriert sind.

Grundsätzlich kann es im Rahmen der vorliegenden Erfindung vorteilhaft und vorgesehen sein, zumindest teilweise die Stromversorgungsanordnung und/oder die Datenübertragungsanordnung in wenigstens einer der wenigstens einen Komponente zu integrieren. In diesem Fall kann die Komponente als zur Videowiedergabeeinrichtung gehörig angesehen werden, oder aber, andersherum, kann die Erfindung auch als auf die entsprechende Komponente gerichtet angesehen bzw. formuliert werden.

In einer besonders bevorzugten Ausgestaltung ist vorgesehen, dass die Komponente ein insbesondere zu der Videowiedergabeeinrichtung gehörendes Kopfkissen ist, in dem die Stromversorgungsanordnung und die Datenübertragungsanordnung wenigstens teilweise integriert sind. In diesem Fall wird also ein Kopfkissen bereitgestellt, in das bereits Teile der Videowiedergabeeinrichtung integriert sind und an dem mittels der Halterung das Anzeigemodul befestigt oder befestigbar sein kann. Insbesondere können hierbei mehrere Halterungen vorgesehen werden, die für entsprechende Lagerungen des Patienten vorgesehen sind. Konkret kann vorgesehen sein, dass die Halterung an einer bezüglich der Längsachse der Patientenliege lateralen Seite des Kopfkissens, insbesondere in einem unteren, plattenartig ausgebildeten Anteil des Kopfkissens, befestigbar ist und/oder dass die Videowiedergabeeinrichtung eine erste Halterung für eine Seitenlage des Patienten zur lateralen Anordnung des Anzeigemoduls in der Verwendungsposition und wenigstens eine zweite Halterung für eine Rückenlage des Patienten zur Anordnung des Anzeigemoduls oberhalb einer Liegefläche des Kopfkissens in der Verwendungsposition aufweist. In einer Ausführungsform können kopfkissenseitige Befestigungsmittel sowohl für die erste als auch für wenigstens eine der wenigstens einen zweiten Halterung verwendet werden. Beispielsweise können gleiche Befestigungspunkte eingesetzt werden. Dies vereinfacht Orientierung, Handhabung und korrekte Anbringung. In der zweckmäßigen Ausgestaltung, in der eine Bewegungseinrichtung, insbesondere Schwenkeinrichtung, vorgesehen ist, kann für die Verwendungsposition insbesondere eine horizontale Stellung eines Halterungsarms der verwendeten Halterung gegeben sein, für die Nichtgebrauchsposition eine horizontale Ausrichtung des Halterungsarms.

In diesem Zusammenhang sieht eine besonders vorteilhafte Weiterbildung vor, dass das Kopfkissen und/oder das Anzeigemodul einen Magnetfeldsensor, insbesondere einen dreidimensionalen Hallsensor, umfasst. Insbesondere kann dabei das Anzeigemodul den Magnetfeldsensor tragen, da dieses klar definiert zum Kopf angeordnet wird, so dass aus Positionsdaten des Magnetfeldsensors leicht auf die Position des Kopfes gefolgert werden kann. Auch ist es denkbar, einen Magnetfeldsensor in einer Baueinheit der Stromversorgungsanordnung und/oder der Datenübertragungsanordnung vorzusehen. Dabei kann zusätzlich oder alternativ eine integrierte Ausbildung mit der Videowiedergabeeinrichtung dadurch erreicht werden, dass der Hallsensor über die Stromversorgungsanordnung bestromt wird und/oder über die Datenübertragungsanordnung kommuniziert.

Kopfkissen für eine Magnetresonanzeinrichtung, in denen ein Magnetfeldsensor, insbesondere ein dreidimensionaler Hall-Sensor, integriert ist, werden beispielsweise durch die nachveröffentlichte deutsche Patentanmeldung mit dem Aktenzeichen 10 2024 205 084.6 beschrieben. Diese dienen dazu, Hinweise auf die Kopfposition des Patienten zu geben, da dies für die Abschätzung der spezifischen Absorptionsrate (SAR), für die spezielle Grenzwerte für den Kopf existieren können, relevant ist. Der Magnetfeldsensor misst dabei insbesondere das Streufeld der Magnetresonanzeinrichtung, welches kennzeichnend für die Position außerhalb der Patientenaufnahme ist. Zuvor wurde bereits vorgeschlagen, in Lokalspulen solche Magnetfeldsensoren zu integrieren, um deren Position zu bestimmen, vergleiche hierzu beispielsweise DE 10 2016 203 255 A1 und US 2017/0248665 A1. Dabei ist es jeweils das Ziel, durch Vermessung des Streufelds eine Position im Koordinatensystem der Patientenliege zu ermitteln. Nachdem bei Bewegungen der Patientenliege deren Art und Ausmaß bekannt ist, folgt daher auch innerhalb der Patientenaufnahme die jeweilige Position, beispielsweise bezüglich des Bildgebungsvolumens.

Nachdem bereits mit der Hinzufügung eines Magnetfeldsensors aus dem Kopfkissen letztlich ein elektronischer Gegenstand wird, ist die Hinzufügung eines Videosystems zu dem Kopfkissen, wie für die erfindungsgemäße Videowiedergabeeinrichtung beschrieben, eine konsequente Weiterbildung, genau wie die Ergänzung des Anzeigemoduls um den Magnetfeldsensor. Gerade im Hinblick auf integrative Aspekte der Produktentwicklung ist eine Kombination aus einem Videosystem, einem Kopfkissen und einem Magnetfeldsensor für die Kopf-Positionsbestimmung (hinsichtlich der SAR) besonders vorteilhaft. Dahingehend kann die vorliegende Erfindung auch als das Kopfkissen betreffend verstanden werden.

Es sind auch Ausführungsbeispiele denkbar, in denen unterschiedliche Kopfkissen für eine Seitenlage und/oder eine Rückenlage verwendet werden. In diesem Fall kann auch vorgesehen sein, in allen Kopfkissen wenigstens teilweise die Stromversorgungsanordnung und/oder die Datenübertragungsanordnung zu integrieren, das bedeutet, diese Komponenten mehrfach vorzusehen.

Eine Befestigung oder Integration ist im Rahmen der vorliegenden Erfindung jedoch auch bezüglich anderer Komponenten, die auf der Patientenliege platziert sind oder werden, denkbar, wobei insbesondere wenigstens dasselbe Anzeigemodul für alle Komponenten verwendet werden kann. So kann beispielsweise vorgesehen sein, dass eine der wenigstens einen Halterung, insbesondere für die Bauchlage des Patienten, zur Befestigung an einem Kopfstützelement und/oder einer Lokalspuleneinrichtung als Komponente vorgesehen ist und/oder wenigstens ein Teil der Stromversorgungsanordnung und/oder der Datenübertragungsanordnung in einer Lokalspuleneinrichtung verbaut ist. Beispielsweise sind mittig offene Kopfstützelemente bekannt, auf die in der Bauchlagerung der Patient seinen Kopf platzieren kann. Auch hier dann kann die oder eine Halterung, insbesondere für die Bauchlage, befestigt sein und/oder es können wenigstens Teile der Datenübertragungsanordnung und/oder der Stromversorgungsanordnung dort integriert sein. Besonders zweckmäßig ist auch die Integration mit bzw. Befestigung an einer Lokalspuleneinrichtung, beispielsweise einer Brustspule, die in manchen Fällen bereits ein soeben diskutiertes Kopfstützelement beinhalten kann. Bei Lokalspuleneinrichtungen kann eine zweckmäßige Weiterbildung vorsehen, dass die Stromversorgung und/oder die Datenversorgung mit wiederzugebenden Bildern über einen Spulenstecker der Lokalspuleneinrichtung erfolgt. In diesem Fall erfolgt also nicht nur die Anbindung der Lokalspuleneinrichtung über den Spulenstecker und den entsprechenden Spulensteckplatz, sondern auch die Anbindung der Videowiedergabeeinrichtung. Hierfür ist es selbstverständlich notwendig, dass die Verbindungen zu den Spulensteckplätzen entsprechend ausgebildet sind. Neben einer Brustspule, die sich hinsichtlich der Bauchlage besonders anbietet, ist es auch denkbar, eine Befestigung und/oder zumindest teilweise Integration mit einer Kopfspule vorzusehen, wobei die Videowiedergabeeinrichtung dann auch in anderweitigen Lagerungen des Patienten eingesetzt werden kann, nachdem die Kopfspule häufig das Kopfkissen ersetzt.

Die Videowiedergabeeinrichtung kann zudem ein Audioausgabemittel umfassen und/oder einem solchen zugeordnet sein. Auch diesbezüglich sind bereits Ausgestaltungen vorgeschlagen worden, bei denen Audioausgabemittel in einem Kopfkissen integriert sind; selbstverständlich können auch grundsätzlich bekannte Kopfhörer eingesetzt werden. Hierbei werden zur Ausgabe bevorzugt Piezoelemente eingesetzt, um möglichst störungsarm zu arbeiten. Durch die Kombination von Video und Audio können beispielsweise Filme, Serien, Musikvideos und dergleichen wiedergegeben werden.

Die vorliegende Erfindung betrifft zudem auch eine Magnetresonanzeinrichtung, aufweisend wenigstens eine Videowiedergabeeinrichtung der erfindungsgemäßen Art. Sämtliche Ausführungen bezüglich der erfindungsgemäßen Videowiedergabeeinrichtung lassen sich analog auf die erfindungsgemäße Magnetresonanzeinrichtung übertragen, sodass auch mit dieser die bereits genannten Vorteile erhalten werden können. Insbesondere kann die Magnetresonanzeinrichtung im Fall der drahtlosen optischen Datenübermittlung die bereits erwähnten Sendeeinrichtungen umfassen. Auch kann eine Steuereinrichtung vorgesehen sein, um Daten auszuwählen bzw. vorzugeben, die mittels der Videowiedergabeeinrichtung und gegebenenfalls parallel dem Audioausgabemittel ausgegeben werden sollen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipskizze einer erfindungsgemäßen Magnetresonanzeinrichtung,
- Fig. 2: ein erstes Ausführungsbeispiel der erfindungsgemäßen Videowiedergabeeinrichtung bei Verwendung einer ersten Halterung,
- Fig. 3: eine Prinzipskizze eines Anzeigemoduls der Videowiedergabeeinrichtung,
- Fig. 4: das erste Ausführungsbeispiel der erfindungsgemäßen Videowiedergabeeinrichtung bei Verwendung einer zweiten Halterung, und
- Fig. 5: ein zweites Ausführungsbeispiel der erfindungsgemäßen Videowiedergabeeinrichtung.

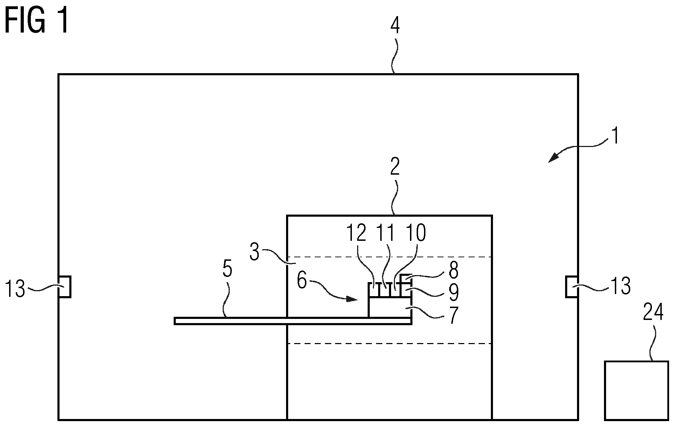

Fig. 1 zeigt eine Prinzipskizze einer erfindungsgemäßen Magnetresonanzeinrichtung 1, von der vorliegend die Hauptmagneteinheit 2, die den supraleitenden Hauptmagneten enthält und eine zylindrische Patientenaufnahme 3 aufweist, näher gezeigt ist. Die Hauptmagneteinheit 2 ist in einer Schirmkabine 4 angeordnet.

Zur Untersuchung kann ein Patient mittels einer Patientenliege 5 in die zylindrische Patientenaufnahme 3 eingefahren werden. Zur Unterhaltung des Patienten ist eine Videowiedergabeeinrichtung 6 vorgesehen, die hier an einer Komponente 7, die auf der Patientenliege 5 anzuordnen oder angeordnet ist, befestigt und/oder mit dieser integriert ist. Die Komponente 7 kann ein Kopfkissen, eine Lokalspuleneinrichtung oder dergleichen sein. Als Teil der Videowiedergabeeinrichtung 6 oder dieser zugeordnet ist zudem ein Audioausgabemittel 8 vorgesehen. Wie hier nur schematisch angedeutet ist, umfasst die Videowiedergabeeinrichtung 6 ein Anzeigemodul 9, eine Halterung 10, eine Stromversorgungsanordnung 11 und eine Datenübertragungsanordnung 12. Hierbei können mehrere Halterungen 10 für unterschiedlichen Komponenten 7 und/oder unterschiedliche Lagerungen eines Patienten vorgesehen sein.

Nachdem die Datenübertragungsanordnung 12 vorliegend beispielhaft zur Nutzung drahtloser optischer Kommunikation ausgebildet ist, sind in der Schirmkabine 4 zwei Sendeeinrichtungen 13 vorgesehen, die in Richtung der Längsachse der Patientenaufnahme 3 und somit der Patientenliege 5 kollimiertes und niederenergetisches Licht zur Li-Fi-Kommunikation und/oder zur Herstellung eines Infrarotlinks aussenden können. Eine der Sendeeinrichtungen 13 sendet von hinten in die Patientenaufnahme 3, die andere von vorne, sodass, egal wie der Patient positioniert ist (Kopf zuerst oder Füße zuerst), einer der Sendeeinrichtungen 13 den Kopfbereich des Patienten und somit die Datenübertragungsanordnung 12, konkret deren Empfangsteil als Schnittstelle, erreicht.

Die Videowiedergabeeinrichtung 6 kann dabei, wie erwähnt und im Folgenden noch genauer erläutert werden wird, mehrere Halterungen 10 umfassen, die unterschiedlichen Lagerungen des Patienten und ggf. auch unterschiedlichen Komponenten zugeordnet sind.

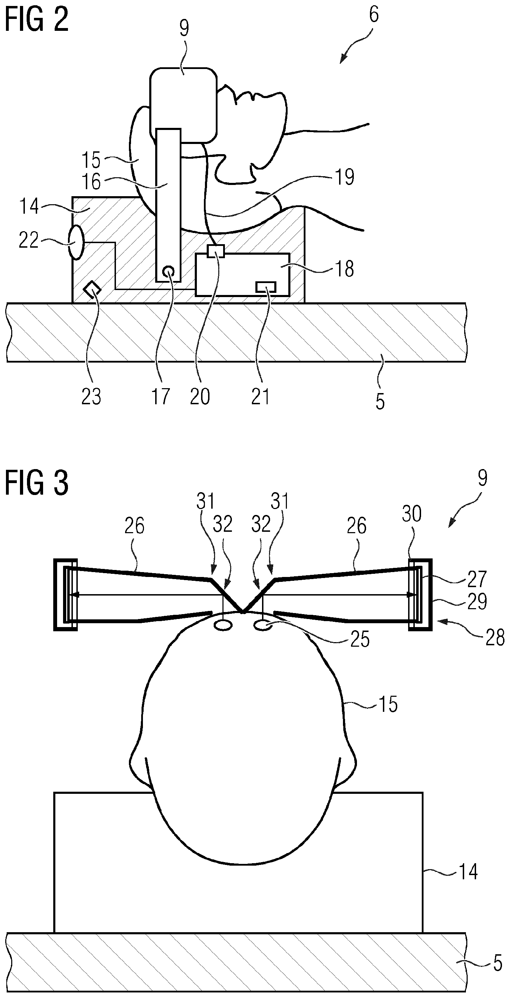

Fig. 2 zeigt ein erstes konkretes Ausführungsbeispiel einer Videowiedergabeeinrichtung 6. Diese ist mit einem Kopfkissen 14 als Komponente 7 integriert ausgebildet bzw. an einem solchen befestigt. Ein Kopf 15 des Patienten ist in Rückenlage auf dem Kopfkissen 14 gezeigt. Die Halterung 10 umfasst vorliegend 2 auf gegenüberliegenden Seiten des Kopfkissens 14 mittels entsprechender Befestigungsmittel (nicht gezeigt) angebrachte Halterungsarme 16, die das Anzeigemodul 9 tragen, welches sich oberhalb des Patientenkopfes 15 vor dessen Augen zwischen den Halterungsarmen 16 erstreckt. Mittels einer Bewegungseinrichtung 17, hier einer Schwenkeinrichtung, können die Halterungsarme 16 mit dem Anzeigemodul 9 oder ohne dieses aus der gezeigten Verwendungsposition, in der sich die Halterungsarme 16 vertikal erstrecken, in eine horizontale, nicht störende Nichtgebrauchsposition verbracht werden. Sowohl in der Verwendungsposition als auch in der Nichtgebrauchsposition ist mittels entsprechender, nicht näher gezeigter Arretierungsmittel eine Arretierung möglich.

Teile der Stromversorgungsanordnung 11 und der Datenübertragungsanordnung 12 sind vorliegend in einer in dem Kopfkissen 14 verbauten Baueinheit 18 realisiert. Die Baueinheit 18 ist über ein Kabel 19 und einen Stecker 20 mit dem Anzeigemodul 9 verbunden. In der Baueinheit 18 ist als Teil der Stromversorgungsanordnung 11 vorliegend ein magnetresonanzkompatibler elektrischer Akkumulator 21, beispielsweise ein Lithium-Ionen-Akkumulator, vorgesehen. Zusätzlich oder alternativ können auch, dann außerhalb der Baueinheit 18 zu verwendende, Photovoltaikmodule als Teil der Stromversorgungsanordnung 11 verwendet werden. Die Datenübertragungsanordnung 12 nutzt hier die optische drahtlose Übertragungstechnik und umfasst daher ein zu wenigstens einer der Sendeeinrichtungen 13 gerichtetes Empfangsteil 22, welches beispielsweise eine Linse und/oder eine Photodiode umfassen kann und mit der Baueinheit 18 verbunden ist. Das Empfangsteil 22 bildet eine Schnittstelle für die drahtlose optische Datenübertragung.

Sobald sich der Patientenkopf 15 auf dem Kopfkissen 14 befindet, wird die Halterung 10 in die vertikale Verwendungsposition verschwenkt und arretiert. Ist das Anzeigemodul zu diesem Zeitpunkt noch nicht an der Halterung 10 befestigt, kann es nun an der Halterung 10 befestigt werden und das Kabel 19 kann mit der Baueinheit 18 verbunden werden. Nun kann die Videowiedergabeeinrichtung 6 mit dem Anzeigen von Bildern, die über die Datenübertragungsanordnung 12 erhalten wurden, beginnen.

Wie Fig. 2 zeigt, umfasst das Kopfkissen, in dieses integriert, vorliegend auch einen Magnetfeldsensor 23, der als dreidimensionaler Hall-Sensor ausgebildet ist. Dieser kann auch über die Stromversorgungsanordnung 11 mit elektrischer Leistung versorgt werden und mittels der Datenübertragungsanordnung 12 seine Messdaten an eine Steuereinrichtung 24 (vergleiche Fig. 1) der Magnetresonanzeinrichtung 1 übertragen, welche umgekehrt auszugebende Bilder als Videodaten und gegebenenfalls zugeordnete Audiodaten für das Audioausgabemittel 8 bereitstellen kann. Das Empfangsteil 22 ist mithin auch zum Senden ausgebildet und die Sendeeinrichtungen 13 auch zum Empfangen. Der Magnetfeldsensor 23 kann in Ausgestaltungen auch Teil des Anzeigemoduls 9 sein.

In einer Alternativvariante können die Datenübertragung und die Stromversorgung bis zum Kopfkissen 14 auch galvanisch über ein Spulenkabel und/oder ein dediziertes Kabelsystem, falls die Spulensteckplätze keine Audio- und Videodatenübertragung bereitstellen, erfolgen. Dies ist jedoch weniger bevorzugt.

Fig. 3 zeigt das Anzeigemodul 9 und dessen Aufbau genauer. Angedeutet ist wiederum der Kopf 15 des Patienten auf dem Kopfkissen 14, wobei der Kopf ebenso lediglich angedeutete Augen 25 umfasst. Obwohl das Anzeigemodul 9 der besseren Erläuterung halber unmittelbar an den Kopf 15 anschließend gezeigt ist, ist es in der Praxis doch beabstandet, insbesondere wenigstens um ein bis zehn cm. Das Anzeigemodul 9 weist vorliegend Anzeigeeinheiten für das rechte und das linke Auge auf, die spiegelsymmetrisch aufgebaut sind und deren Trägeranordnungen 26 (hier nur angedeutet) durch ein Verbindungsmittel (nicht gezeigt), welches lösbar sein kann, zu einer gemeinsamen Trägereinrichtung verbunden sind. Jede Anzeigeeinheit weist zunächst eine Displayeinrichtung 27 auf, die beispielsweise als LCD, OLED-Display oder Mikro-LED-Display ausgebildet sein kann. Die Displayeinrichtung 27 ist von einem Hochfrequenzschirm 28 umgeben, der vorliegend zwei Anteile aufweist. Der nicht die Displayfläche der Displayeinrichtung 27 überdeckende Anteil 29 besteht aus mehreren Kohlenstofffaserschichten, zwischen denen jeweils eine Gitterstruktur bzw. Gitterschicht aus Kupfer eingebettet ist, deren Maschenbreite beispielsweise ein bis vier mm betragen kann. Der Anteil 29 ist also eine Schichtstruktur. Auf der Seite der Displayfläche wird der Hochfrequenzschirm 28 durch mit ITO beschichtetes Glas 30 in mehreren Glasschichten hochfrequenzdicht ausgestaltet. Jede ITO-Schicht ist entlang des Randes galvanisch mit dem Anteil 29 verbunden.

Auch für die Stromversorgungsanordnung 11 und die Datenübertragungsanordnung 12 können möglicherweise die Magnetresonanzbildgebung störende Anteile mit einem solchen Hochfrequenzschirm 28, dann in der Ausgestaltung des Anteils 29 als Schichtstruktur, vorgesehen sein.

Nachdem die Displayeinrichtungen 27 seitlich, insbesondere in einem 90°-Winkel zur Blickrichtung, angeordnet sind, weist jede Anzeigeeinheit eine Optikanordnung 31 auf, die vorliegend wenigstens einen parabolischen, halbtransparenten, zumindest im Wesentlichen in einem 45°-Winkel stehenden Spiegel 32 umfasst. Zusätzlich kann die Optikanordnung 31 jeweils auch eine hier nicht näher dargestellte Linse umfassen, beispielsweise zur Fokussierung. Wie zu erkennen ist, wird der optische Pfad mithilfe der Spiegel 32 und gegebenenfalls weiterer Optik in eine horizontale Richtung umgelenkt und endet bei den Displayeinrichtungen 27 links und rechts vom Kopf 15 des Patienten. So ist ein flacher Aufbau bezüglich der vertikalen Achse gegeben, sodass die Bewegungsfreiheit der Patientenliege 5 in horizontaler Richtung nicht eingeschränkt wird. Zum anderen befinden sich die beiden Displayeinrichtungen 27 relativ weit entfernt, beispielsweise wenigstens 5 cm entfernt, vom Kopf 15 des Patienten, sodass Bildartefakte vermieden werden können. Durch den (hier nicht genauer gezeigten) Abstand des Anzeigemoduls 9 von den Augen 25 und die teilweise transparente Ausgestaltung der Spiegel 32 ist der Patient nicht vollständig von der Umgebung abgeschottet.

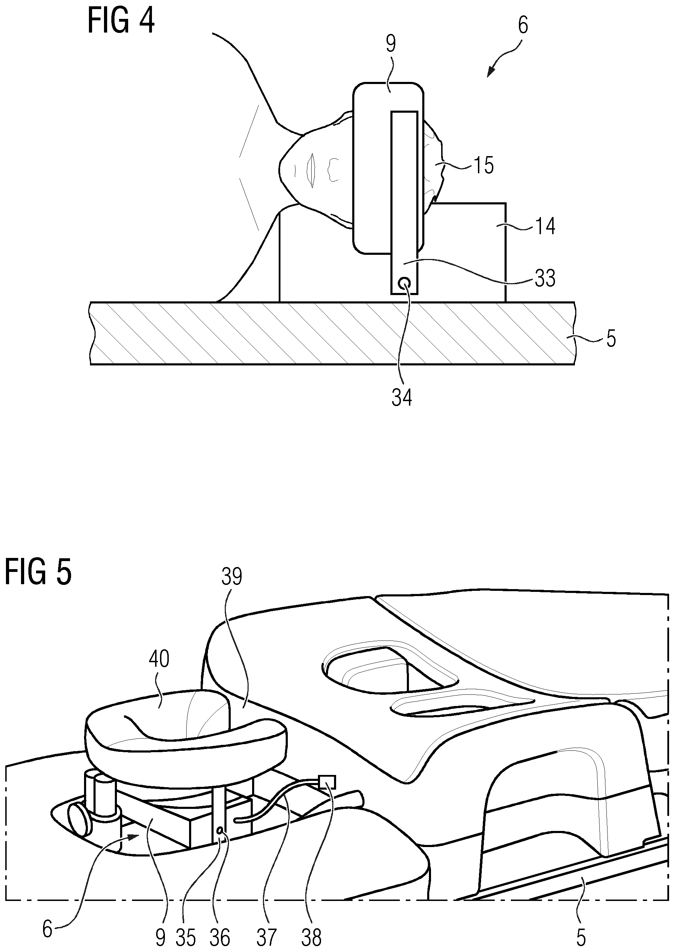

Fig. 4 erläutert, wie unter Verwendung einer anderen Halterung 10 mit einem Halterungsarm 33 und einer Bewegungseinrichtung 34 die Videowiedergabeeinrichtung 6 auch bei einer Seitenlage des Patienten genutzt werden kann. Die Fig. 4 erläutert dabei die Seitenlage "links", für die Seitenlage "rechts" kann eine entsprechende Anordnung auf der anderen Seite des Kopfkissens 14 verwendet werden. Wiederum ist die Verwendungsposition gezeigt; für die Nichtgebrauchsposition kann der Halterungsarm 33 in die Vertikale geschwenkt werden.

Fig. 5 zeigt schließlich ein zweites Ausführungsbeispiel einer erfindungsgemäßen Videowiedergabeeinrichtung 6 mit einem Anzeigemodul 9, das wie in Fig. 3 gezeigt ausgebildet ist, und einer durch einen Halterungsarm 35 und Befestigungsmittel 36 für das Anzeigemodul 9 gebildeten Halterung 10, wobei die Stromversorgungsanordnung 11 und die Datenübertragungsanordnung 12 vorliegend durch ein Kabel 37 mit einem Stecker 38 gebildet werden, die an einer Brustspule 39 als Komponente 7 angeschlossen sind. Die Versorgung mit Videodaten und elektrischer Leistung erfolgt in diesem Fall also über die in einen Spulensteckplatz eingesteckte Brustspule 39. Es ist jedoch auch in diesem Ausführungsbeispiel eine optische drahtlose Übertragung grundsätzlich möglich. Zweckmäßigerweise können weitere Komponenten der Stromversorgungsanordnung 11 und der Datenübertragungsanordnung 12 innerhalb der Brustspule 39 integriert sein.

Die Brustspule 39 umfasst vorliegend auch ein Kopfstützelement 40, an dessen Träger der Halterungsarm 35 mittels entsprechender Befestigungsmittel befestigt ist. Die Ausgestaltung der Fig. 5 eignet sich für die Bauchlage des Patienten.

*Unabhängig vom grammatikalischen Geschlecht eines bestimmten Begriffes sind Personen mit männlicher, weiblicher oder anderer Geschlechteridentität mit umfasst.*

## Patentansprüche

1. Modulare Videowiedergabeeinrichtung (6) für einen Patienten auf einer Patientenliege (5) in einer Magnetresonanzeinrichtung (1), aufweisend
- wenigstens ein Anzeigemodul (9) mit jeweils einer Anzeigeeinheit für ein linkes und ein rechtes Auge (25) eines Kopfes (15) des Patienten, wobei jede Anzeigeeinheit umfasst
* eine Displayeinrichtung (27),
* eine Trägeranordnung (26), um die Displayeinrichtung (27) in einer von dem Kopf (15), insbesondere seitlich, beabstandeten Position bei in einer Verwendungsposition befindlicher Anzeigeeinheit zu haltern, und
* eine an der Trägeranordnung (26), insbesondere wenigstens teilweise vor dem jeweiligen Auge (25), befestigte Optikanordnung (31) zur Projektion eines von der Displayeinrichtung (27) ausgegebenen Bildes in der Verwendungsposition zu dem Auge (25),
- wenigstens eine Halterung (10), die mit dem Anzeigemodul (9) lösbar verbindbar ist und Befestigungsmittel zur lösbaren Befestigung der Halterung (10) wenigstens an einer auf der Patientenliege (5) angeordneten und/oder anordenbaren Komponente (7) aufweist,
- eine mit dem Anzeigemodul (9) verbindbare Datenübertragungsanordnung (12), und
- eine mit dem Anzeigemodul (9) verbindbare Stromversorgungsanordnung (11).

2. Videowiedergabeeinrichtung nach Anspruch 1, **dadurch gekennzeichnet, dass** sie mehrere austauschbare Halterungen (10) für unterschiedlichen Liegepositionen des Patienten zugeordnete Komponenten (7) und/oder Befestigungspositionen an wenigstens einer der wenigstens einen Komponente (7) aufweist und/oder dass die Trägeranordnungen (26) beider Anzeigeeinheiten zu einer Trägereinrichtung des Anzeigemoduls (9) mittels eines Verbindungsmittels verbindbar oder verbunden sind.

3. Videowiedergabeeinrichtung nach Anspruch 1 oder 2, **dadurch gekennzeichnet, dass** wenigstens eine der wenigstens einen Halterung (10) eine Bewegungseinrichtung (17, 34), insbesondere eine Schwenkeinrichtung, zum Bewegen des Anzeigemoduls (9) zwischen der Verwendungsposition und wenigstens einer Nichtgebrauchsposition aufweist.

4. Videowiedergabeeinrichtung nach einem der vorangehenden Ansprüche, **dadurch gekennzeichnet, dass** die Stromversorgungsanordnung (11) einen magnetresonanzkompatiblem elektrischen Akkumulator (21) und/oder wenigstens ein Photovoltaikmodul und/oder wenigstens ein Kabel (37) und/oder wenigstens einen Stecker (38) umfasst.

5. Videowiedergabeeinrichtung nach einem der vorangehenden Ansprüche, **dadurch gekennzeichnet, dass** die Datenübertragungsanordnung (12) eine Schnittstelle zum optischen Empfang wiederzugebender Bilder, insbesondere einen Anschluss für ein Glasfaserkabel und/oder eine LiFi-Schnittstelle und/oder eine Infrarotlink-Schnittstelle, aufweist.

6. Videowiedergabeeinrichtung nach einem der vorangehenden Ansprüche, **dadurch gekennzeichnet, dass** die Anzeigeeinheiten, insbesondere wenigstens deren Displayeinrichtungen (27), und/oder die Stromversorgungsanordnung (11) und/oder die Datenübertragungsanordnung (12) einen Hochfrequenzschirm (28) aufweisen.

7. Videowiedergabeeinrichtung nach Anspruch 6, **dadurch gekennzeichnet, dass** der Hochfrequenzschirm (28) wenigstens teilweise eine Schichtstruktur umfasst, in der zwischen zwei Kohlenstofffaserschichten eine metallische, insbesondere aus Kupfer bestehende Gitterschicht angeordnet ist.

8. Videowiedergabeeinrichtung nach Anspruch 6 oder 7, **dadurch gekennzeichnet, dass** der Hochfrequenzschirm (28) der Displayeinrichtungen (27) eine Displayfläche der jeweiligen Displayeinrichtung (27) überdeckend wenigstens eine mit ITO und/oder Silber und/oder Gold beschichtete Glasschicht (30) aufweist, insbesondere mehrere solche Glasschichten (30).

9. Videowiedergabeeinrichtung nach einem der vorangehenden Ansprüche, **dadurch gekennzeichnet, dass** die Stromversorgungsanordnung (11) und die Datenübertragungsanordnung (12) wenigstens teilweise als eine gemeinsame Baueinheit (18), insbesondere ein Baueinheitmodul, in einem gemeinsamen Gehäuse angeordnet sind.

10. Videowiedergabeeinrichtung nach einem der vorangehenden Ansprüche, **dadurch gekennzeichnet, dass** die Komponente (7) ein insbesondere zu der Videowiedergabeeinrichtung (6) gehörendes Kopfkissen (14) ist, in dem die Stromversorgungsanordnung (11) und die Datenübertragungsanordnung (12) wenigstens teilweise integriert sind.

11. Videowiedergabeeinrichtung nach Anspruch 10, **dadurch gekennzeichnet, dass** die Halterung (10) an einer bezüglich der Längsachse der Patientenliege (5) lateralen Seite des Kopfkissens (14) befestigbar ist und/oder eine erste Halterung (10) für eine Seitenlage des Patienten zur lateralen Anordnung des Anzeigemoduls (9) in der Verwendungsposition und wenigstens eine zweite Halterung (10) für eine Rückenlage des Patienten zur Anordnung des Anzeigemoduls (9) oberhalb einer Liegefläche des Kopfkissens (14) in der Verwendungsposition aufweist.

12. Videowiedergabeeinrichtung nach Anspruch 10 oder 11, **dadurch gekennzeichnet, dass** das Kopfkissen (14) und/oder das Anzeigemodul (9) einen Magnetfeldsensor (23), insbesondere einen dreidimensionalen Hallsensor, umfasst.

13. Videowiedergabeeinrichtung nach einem der vorangehenden Ansprüche, **dadurch gekennzeichnet, dass** eine der wenigstens eine Halterung (10), insbesondere für die Bauchlage des Patienten, zur Befestigung an einem Kopfstützelement (40) und/oder einer Lokalspuleneinrichtung als Komponente (7) vorgesehen ist und/oder wenigstens ein Teil der Stromversorgungsanordnung (11) und/oder der Datenübertragungsanordnung (12) in einer Lokalspuleneinrichtung verbaut ist.

14. Videowiedergabeeinrichtung nach einem der vorangehenden Ansprüche, **dadurch gekennzeichnet, dass** sie zudem ein Audioausgabemittel (8) umfasst und/oder einem solchen zugeordnet ist.

15. Magnetresonanzeinrichtung (1), aufweisend eine Videowiedergabeeinrichtung (6) nach einem der vorangehenden Ansprüche.
